# EUROPEAN PATENT SPECIFICATION

(11) **EP 0 601 845 B1**
(45) Date of publication and mention of the grant of the patent: **09.09.1998**
(21) Application number: 93309875.8
(22) Date of filing: 08.12.1993
(51) Int. Cl.: G02C 7/04, G02C 7/06

(54) **Multifocal ophthalmic lens pair**
Multifokales Linsenpaar zur Korrektur von Sehfehlern
Paire de lentilles ophthalmiques multifocales

(30) Priority: 09.12.1992 US 988071
(43) Date of publication of application: 15.06.1994
(73) Proprietor: JOHNSON & JOHNSON VISION PRODUCTS, INC., Jacksonville, Florida 32216 (US)
(72) Inventor: Roffman, Jeffrey H., Jacksonville, FL 32223 (US); Poling, Timothy R., Jacksonville, FL 32223 (US); Guillon, Michel, London SW1 V3EY (GB); Menezes, Edgar, Jacksonville, FL 32216 (US)
(74) Representative: Mercer, Christopher Paul

(56) References cited:
- EP-A- 0 107 444
- EP-A- 0 445 994
- EP-A- 0 453 136
- US-A- 4 923 296
- US-A- 5 024 517
- US-A- 5 056 909
- US-A- 5 151 723

## Description

### BACKGROUND OF THE INVENTION

This invention pertains to the subject of ophthalmic lenses, and in particular contact lenses containing more than one optical power or focal length.

It is well known that as an individual ages, the eye is less able to accommodate, i.e., bend the natural lens in the eye in order to focus on objects that are relatively near to the observer. This condition is referred to as presbyopia, and presbyopes have in the past relied upon spectacles or other lenses having a number of different regions with different optical powers to which the wearer can shift his vision in order to find the appropriate optical power for the object or objects upon which the observer wishes to focus.

With spectacles this process involves shifting one's field of vision from typically an upper, far power to a different, near power. With contact lenses, however, this approach has been less than satisfactory. The contact lens, working in conjunction with the natural lens, forms an image on the retina of the eye by focusing light incident on each part of the cornea from different field angles onto each part of the retina in order to form the image. This is demonstrated by the fact that as the pupil contracts in response to brighter light, the image on the retina does not shrink, but rather, light coming through a smaller area of the lens constructs the entire image.

Similarly, for a person that has had the natural lens of the eye removed because of a cataract condition and an intraocular lens inserted as a replacement, the ability to adjust the lens (accommodate) to the distance of the object being viewed is totally absent. In this case, the lens provided is usually set at the singe infinity distance focal power and spectacles are worn to provide the additional positive optical power needed for in-focus close vision. For such a patient, a functional multifocal lens would be particularly useful.

It is known in the art that under certain circumstances that the brain can discriminate separate competing images by accepting the in-focus image and rejecting the out-of-focus image.

One example of this type of lens used for the correction of presbyopia by providing simultaneous near and far vision is described in U.S. 4,923,296 to Erickson. Described therein is a lens system which comprises a pair of contact lenses each having equal areas of near and distant optical power, the lens for one eye with a near upper half and a distant lower half while the lens for the other eye contains a distant upper half and near lower half. Together these are said to provide at least partial clear images in both eyes, and through suppression by the brain of the blurred images, allows alignment of the clear image to produce an in-focus image.

U.S. Patent number 4,890,913 to de Carle describes a bifocal contact lens comprising a number of annular zones having different optical powers. The object in the design of this lens is to maintain, at all times regardless of pupil diameter, an approximately equal division between near and distant powers, requiring on the lens between 6 and 12 total zones.

Another attempt at providing a bifocal contact lens is described in U.S. Patent number 4,704,016 to de Carle. Again, this lens attempts to maintain, at all times regardless of pupil diameter, an approximately equal division between near and distant powers.

Another approach to producing a multifocal corrective eye lens involves the use of diffractive optics. One of the shortcomings of this approach has been a deficiency in vision at low light levels. In a diffractive design only about 40% of the light incident on the lens is used for near vision with another 40% being used for far vision. The remaining 20% is not used for either near or far vision, but rather is lost to higher orders of diffraction and scatter effect. This represents the best theoretical case and in manufacturing reality even less light is available due to manufacturing difficulties. Difficulty of manufacture in general represents another shortcoming of diffractive lenses since the diffractive surface must be to tolerances on the order of the wavelength of light.

One attempt known in the art to provide a method of compensating for presbyopia without complex lens manufacture is known as "monovision". In the monovision system a patient is fitted with one contact lens for distant vision in one eye and a second contact lens for near vision in the other eye. Although it has been found that with monovision a patient can acceptably distinguish both distance and near objects, there is a substantial loss of binocularity, i.e. depth perception.

In EP-A-0453136 there is disclosed a method of correcting or alleviating presbyopia in humans which comprises fitting a first contact lens to a first eye, and fitting a second contact lens to the other eye. Each of the lenses has a first power provided by the shape, curvature and material of the lens to provide a refractive corrected image and a second power provided by diffractive means to provide a second corrected image, the second power being additive to (or subtracted from) the first power. The lenses are fitted so that one has a refractive near image optical power and the other has a refractive distance image optical power. The diffractive means of the lenses are arranged so that the amount of the light directed into the near image by one lens is substantially greater than that directed into the far image by that lens, and the amount of light directed into the far image by the other lens is substantially greater than that directed into the near image by that other lens. In this way the wearer sees an image of relatively high intensity and retains a large amount of stereoscopic vision.

Although simple systems such as monovision are somewhat understood, more complex schemes for multifocal refractive lenses are primarily theoretical.

U.S. Patents 5,002,382 and 5,024,517 both issued to Seidner, disclose complementary pairs of contact lenses having two or more corrective optical powers in opposite configurations. Both of the lens pairs described are closely related to the monovision concept: the pair has a central power disparity between lenses, and fails to effect a power ratio adjustment as a function of illumination.

European Patent Application, Publication number 0 201 231 A2 by Ho, et al. describes a binocular pair of contact lenses having a plurality of optical zones that are complementary between lenses, i.e. opposite alternate zones of near and distance powers.

A more practical and improved approach to providing a multi-focal ophthalmic lens is described in European Patent Application, Publication Number 0 553 959. In this application there is disclosed a multifocal ophthalmic lens characterized by having a central zone wherein one of the multifocal segments includes the central zone of the lens. The boundary between the segments is defined by an arcuate path such as a semicircle having both ends of the path on the adjoining parameter of the near and distant segments to eliminate from the central optical axis the segment boundaries including the central junction point.

While the lenses made according to the above described applications are functional and the manufacturing techniques described therein are a practical way of molding ophthalmic lenses, an important aspect of proper multifocal vision under various light conditions has not been met.

Under real-world conditions, the ability of the brain to discriminate between (or among) two or more images where only one is in-focus is greatly enhanced if the central part of the image consists of only one focal length. It has also been found that high levels of illumination typically are accompanied by distance vision situations.

It is an object, therefore, of the present invention to provide a pair of ophthalmic lenses for a presbyope that yields improved visual acuity in general, and particularly under high intensity light conditions.

It is a further object of the invention to describe a method for determining the manner in which such lenses are to be fitted to a patient to produce the desired improvement in vision, especially by matching the optical power required for a high illumination situation.

### SUMMARY OF THE INVENTION

In accordance with the present invention, there is provided a method of providing multifocal vision correction to a patient as specified in claim 1 hereinafter.

Also in accordance with the present invention, there is provided a pair of ophthalmic lenses as specified in claim 2 hereinafter.

The above objects are achieved by providing a pair of ophthalmic lenses both containing at least two optical powers, one for near vision and one for distance vision.

Both lenses, however, contain in the center portion of the lens the distant power. In the preferred embodiment, the remainder of the lens is comprised of annular portions each made of one or more optical zones to provide the desired combined, cumulative ratio of near and distance focal length areas at each pupil diameter.

In this way, the center portion of the vision contains a single optical power which results in improved visual acuity. This distance portion in the center is particularly well suited to the real world situation of requiring distance vision under high illumination situations.

### BRIEF DESCRIPTION OF THE DRAWINGS

Figure 1 shows the optical zone of a pair of ophthalmic lenses constructed according to the principles of the present invention.

Figure 2 is a bar graph comparing the fraction of available light striking the pupil for near and distant focal lengths for the ophthalmic lens pair of Figure 1 as a function of pupil diameter.

### DESCRIPTION OF THE PREFERRED EMBODIMENT

As is well known in the art, people have a dominant eye. This eye can be found by having the patient look through an opaque sheet with a hole in it. The patient is asked to sight a distant object by looking through the hole. When properly arranged, the hole in the sheet, the size of the hole and the distance from the patient's eye allows the distant object to be seen through the hole in the sheet by only one eye at a time.

After the patient has sighted the object through the hole, the eyes are alternately covered to determine which eye has been used to sight the object, and is therefore, the dominant eye. In most people, the right eye is the dominant eye.

It has been found that a crucial characteristic in the construction of a multifocal ophthalmic lens needed to attain visual acuity, is that a single central focal length be maintained and disparity in the central visual region be avoided. Although the importance of this in a single lens was recognized in my prior above-referenced patent application (wherein the central zone of a single lens maintains a single optical power without a boundary or junction point between different optical powers of a multifocal lens), it has now been recognized that it is important to establish a single optical power on the two lenses placed in the eyes of a patient.

It has been found, as described in EP 0 601 846 A "Pupil-Tuned Multifocal Ophthalmic Lens," that in real world conditions, high intensity illumination situations generally are accompanied by the need to focus at a distance.

For this reason the lens pair of the present invention contains at its central portion in both lenses the distant optical power required by the patient.

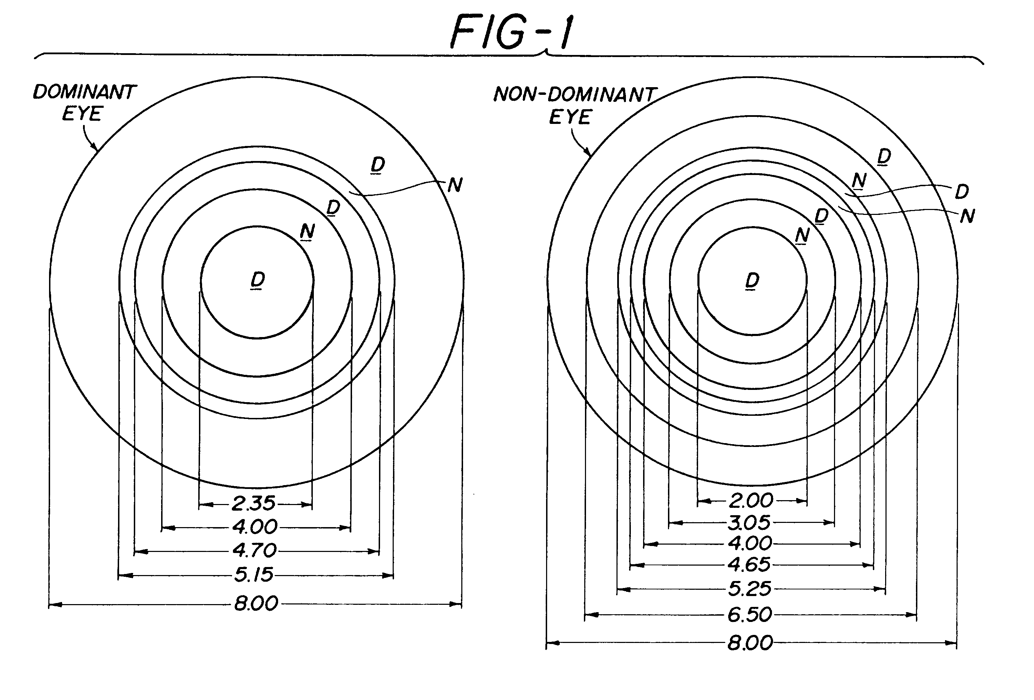

Referring to Figure 1 there is shown the optical zone of a pair of ophthalmic lenses constructed according to the present invention. The typical contact lens is usually constructed with a non-optical lenticular area (not shown) outside the optical surface for a total diameter of 14 mm.

A comparison of the annular zones of the two lenses reveals the important aspects of the present invention. First, both lenses contain a central zone that has the distant optical power for the patient. Second, the remaining zones of both lenses combined contain cumulative near and distant powers to yield the desired ratio of near and distance powers.

The following Table I shows the actual specifications for the distribution of the near and distant power as a function of the diameter through the center of the optical axis.

**TABLE I**

| BINOCULAR SET-ZONE TRANSITIONS | | | | |
|---|---|---|---|---|
| PUPIL DIAMETER | DOMINANT EYE | | NON-DOMINANT EYE | |
| | Distance | Near | Distance | Near |
| 0.00 | 100 | 0 | 100 | 0 |
| 0.50 | 100 | 0 | 100 | 0 |
| 1.00 | 100 | 0 | 100 | 0 |
| 1.50 | 100 | 0 | 100 | 0 |
| 2.00 | 100 | 0 | 0 | 100 |
| 2.35 | 0 | 100 | 0 | 100 |
| 2.50 | 0 | 100 | 0 | 100 |
| 3.00 | 0 | 100 | 0 | 100 |
| 3.05 | 0 | 100 | 100 | 0 |
| 3.50 | 0 | 100 | 100 | 0 |
| 4.00 | 100 | 0 | 0 | 100 |
| 4.50 | 100 | 0 | 0 | 100 |
| 4.65 | 100 | 0 | 100 | 0 |
| 4.70 | 0 | 100 | 100 | 0 |
| 5.00 | 0 | 100 | 100 | 0 |
| 5.15 | 100 | 0 | 100 | 0 |
| 5.25 | 100 | 0 | 0 | 100 |
| 5.50 | 100 | 0 | 0 | 100 |
| 6.00 | 100 | 0 | 0 | 100 |
| 6.50 | 100 | 0 | 100 | 0 |
| 7.00 | 100 | 0 | 100 | 0 |
| 7.50 | 100 | 0 | 100 | 0 |
| 8.00 | 100 | 100 | 100 | 0 |

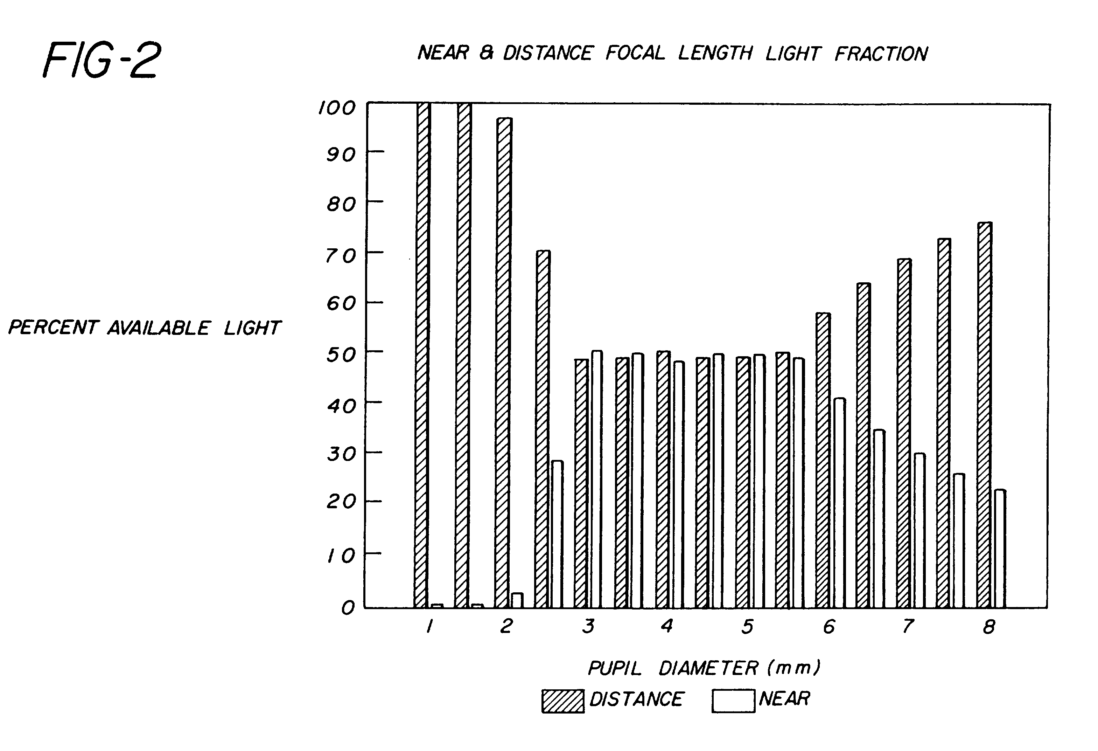

The advantage of a lens constructed according to the above design is readily apparent from Figure 2.

While both lenses of the pair have a center portion in the optical zone that is dedicated to distant vision (the power determined to be required under most real-world conditions), Figure 2 shows as the pupil diameter increases, the light going through the pupil becomes evenly divided between near and distant focal lengths. This provides adequate viewing at both near and far distances as the available light diminishes.

The following Table II shows numerically for the dominant eye lens and non-dominant eye lens, the ratio between the area at each pupil diameter of the surface devoted to the distant focal length and the near focal length.

In addition, the third part of this Table shows the sum, on a percentage basis, for both lenses in the pair for distant and near surface area.

**TABLE II**

| FOCAL LENGTH RATIOS | | | | | | |
|---|---|---|---|---|---|---|
| | DOMINANT EYE | | NON-DOMINANT EYE | | BINOCULAR SUM | |
| | cumulative area (mm)² | | | | **%** of total area | |
| Diameter | Distance | Near | Distance | Near | Distance | Near |
| 1 | 0.8 | 0.0 | 0.8 | 0.0 | 100 | 0 |
| 2 | 3.1 | 0.0 | 3.0 | 0.2 | 97.5 | 2.5 |
| 3 | 4.2 | 2.9 | 3.0 | 4.1 | 50.5 | 49.5 |
| 4 | 4.5 | 8.1 | 8.2 | 4.4 | 50.3 | 49.7 |
| 5 | 8.9 | 10.8 | 11.2 | 8.4 | 51.1 | 48.9 |
| 6 | 16.7 | 11.5 | 12.8 | 15.5 | 52.2 | 47.8 |
| 7 | 26.9 | 11.5 | 18.6 | 19.9 | 59.2 | 40.8 |
| 8 | 38.7 | 11.5 | 30.4 | 19.9 | 68.7 | 31.3 |

A comparison of the available light that passes through the distant and near optical zones of the lens that actually enters the pupil of the eye shows that at high illumination levels (when the pupil of the eye is contracted to admit only a small percentage (less than 10%) of the available light through the pupil of the eye), nearly all of it passes through the distant optical zones contained on the pair of lenses.

As the light diminishes and the pupil diameter increases, beginning at approximately 3 mm pupil diameter, it can be seen that the ratio on a cumulative basis of distance focal area to near focal area then becomes approximately equal between the distant and near surface area for light actually entering through the pupil and into the eye.

As an alternative to using concentric annular optical zones, the annular portions may have the requisite ratio of distant and near focal length areas by employing the design scheme found in European Patent Application, Publication Number 0 553 959. This design method employs continuous radial segments containing different optical powers across annular portions.

As a further improvement to the specific execution of this lens design, it may be preferred to incorporate the teachings of my earlier U.S. Patent 5,505,981, in the design of the surface of the peripheral zone of the non-dominant eye lens. That is, the incorporation of an aspheric lens design on the near vision portion of the lens containing a peripheral near optical zone.

Other variations on the above described invention are possible by the incorporation of aspheric and the combination of aspheric and spherical on other lens surfaces, but do not depart from the limitations of the invention which are given in the following claims.

## Claims

1. A method of providing multifocal vision correction to a patient, said method comprising the steps of:
determining the dominant eye of the patient,
determining the near optical power required for the patient,
determining the distance optical power required for the patient,
providing the patient with a pair of lenses having optical surfaces, both lenses containing zones having only the near optical power, and zones having only the distance optical power, with distance optical power at a center zone of both lenses, wherein the area of said determined distance optical power of the dominant eye lens amounts to more than 50% of the total lens optical surface and wherein the area of said determined near optical power of the non-dominant eye lens amounts to more than 50% of the total lens optical surface.

2. A pair of opthalmic lenses having optical surfaces, both lenses containing zones having only a near optical power, and zones having only a distance optical power, with the distance optical power at a center zone of both lenses, one lens of said pair having an area of said distance optical power amounting to more than 50% of the total lens optical surface and the other lens of said pair having an area of said near optical power amounting to more than 50% of the total lens optical surface.

3. The lens of claim 2 wherein the lenses of said lens pair are of an annular, concentric zone construction.

4. The lens pair of claim 2 wherein between lenses the zones, exclusive of the center zone, have complementary placement of distance optical power and near optical power.

5. The lens pair of claim 2 further comprising an annular portion exterior said center zone containing more near optical power area than distance optical power area such that the total combined near and distance area of the center zone and annular portion are substantially equal.

## Patentansprüche

1. Verfahren zur Erzielung einer multifokalen Sichtkorrektur bei einem Patienten mit den Schritten:
Bestimmung des dominierenden Auges des Patienten;
Bestimmung der für den Patienten erforderlichen Nahbrechkraft,
Bestimmung der für den Patienten erforderlichen Fernbrechkraft,
Ausstattung des Patienten mit einem Linsenpaar mit optischen Flächen, wobei beide Linsen Zonen nur mit Nahbrechkraft und solche nur mit Fernbrechkraft aufweisen und die Fernbrechkraft sich in der Zentralzone beider Linsen befindet und bei welchen die Fläche der bestimmten Fernbrechkraft des dominierenden Auges mehr als 50 % sowie die Fläche der bestimmten Nahbrechkraft des nicht dominierenden Auges mehr als 50 % der gesamten optischen Fläche der Linsen ausmacht.

2. Ophthalmisches Linsenpaar mit optischen Flächen, wobei beide Linsen Zonen nur mit Nahbrechkraft und solche nur mit Fernbrechkraft aufweisen und die Fernbrechkraft sich in der Zentralzone beider Linsen befindet und bei welchem eine Linse des Paares eine Fläche der bestimmten Fernbrechkraft von mehr als 50 % der gesamten optischen Fläche der Linse und die andere Linse des Paares eine Fläche der bestimmten Nahbrechkraft von mehr als 50 % der gesamten optischen Fläche der Linsen aufweist.

3. Linse nach Anspruch 2, bei welchen das Linsenpaar ringförmige, konzentrische Zonen aufweist.

4. Linsenpaar nach Anspruch 2, bei welchem die Linsen untereinander mit Ausnahme der Zentralzone eine komplementäre Anordnung von Fernbrechkraft und Nahbrechkraft aufweisen.

5. Linsenpaar nach Anspruch 2, welches weiterhin einen Ringbereich außerhalb der Zentralzone mit mehr Nahbrechkraftfläche als Fernbrechkraftfläche aufweist, derart, daß die gesamte zusammengefaßte Nah- und Fernbrechkraftfläche der Zentralzone und des Ringbereiches im wesentlichen gleich sind.

## Revendications

1. Procédé permettant la correction multifocale de la vision d'un patient, ledit procédé comprenant les étapes consistant à :
déterminer l'oeil dominant du patient ;
déterminer la puissance optique en vision de près nécessaire au patient ;
déterminer la puissance optique en vision de loin nécessaire au patient ;
doter le patient d'une paire de lentilles possédant des surfaces optiques, l'une et l'autre lentille contenant des zones ayant uniquement la puissance optique pour la vision de près et des zones ayant uniquement la puissance optique pour la vision de loin, la puissance optique pour la vision de loin étant située dans une zone centrale des deux lentilles ;
procédé dans lequel l'aire de surface de ladite puissance optique déterminée pour la vision de loin dans la lentille de l'oeil dominant est égale à plus de 50 % de la surface optique totale de la lentille, et dans lequel l'aire de surface de ladite puissance optique déterminée pour la vision de près dans la lentille de l'oeil non dominant est égale à plus de 50 % de la surface optique totale de la lentille.

2. Paire de lentilles ophtalmiques ayant des surfaces optiques, l'une et l'autre lentille contenant des zones ayant uniquement une puissance optique pour la vision de près et des zones ayant uniquement une puissance optique pour la vision de loin, la puissance optique pour la vision de loin étant située dans une zone centrale des deux lentilles, une lentille de ladite paire ayant une aire de surface de ladite puissance optique pour la vision de loin égale à plus de 50 % de la surface optique totale de la lentille, et l'autre lentille de ladite paire ayant une aire de surface de ladite puissance optique pour la vision de près égale à plus de 50 % de la surface optique totale de la lentille.

3. Lentille selon la revendication 2, dans laquelle les lentilles de ladite paire sont réalisées avec des zones annulaires concentriques.

4. Paire de lentilles selon la revendication 2, dans laquelle les zones, à l'exclusion de la zone centrale, ont un placement de la puissance optique pour la vision de loin et de la puissance optique pour la vision de près complémentaire entre les lentilles.

5. Paire de lentilles selon la revendication 2, comprenant en plus une partie annulaire extérieure à ladite zone centrale et contenant une surface de puissance optique pour la vision de près plus grande que la surface optique pour la vision de loin de sorte que le total combiné des surfaces pour la vision de près et de loin de la zone centrale et celui de la partie annulaire sont sensiblement égaux.
